# EUROPÄISCHE PATENTSCHRIFT

(11) **EP 3 148 504 B1**
(45) Veröffentlichungstag und Bekanntmachung des Hinweises auf die Patenterteilung: **18.07.2018**
(21) Anmeldenummer: 15721683.9
(22) Anmeldetag: 06.05.2015
(51) Int. Cl.: A61K 8/37, A61K 8/60, A61Q 9/02, A61K 8/06

(54) **VERFAHREN ZUR HERSTELLUNG EINES KONDITIONIERENDEN PRESHAVE- ODER AFTERSHAVEMITTELS**
METHOD TO PRODUCE A CONDITIONING PRESHAVE OR AFTERSHAVE COMPOSITION
METHODE POUR LA PRODUCTION D'UNE COMPOSITION PRE OU APRÉS RASAGE

(30) Priorität: 30.05.2014 DE 102014210275
(43) Veröffentlichungstag der Anmeldung: 05.04.2017
(73) Patentinhaber: Henkel AG & Co. KGaA, 40589 Düsseldorf (DE)
(72) Erfinder: KAMPMANN, Martina, 41352 Korschenbroich (DE); HEIDE, Barbara, 47809 Krefeld (DE)
(86) Internationale Anmeldenummer: PCT/EP2015/059895
(87) Internationale Veröffentlichungsnummer: WO 2015/180938

(56) Entgegenhaltungen:
- EP-A2- 0 507 047
- EP-A2- 0 813 862
- WO-A1-2009/051486
- WO-A2-2012/130655
- US-A1- 2011 262 370

## Beschreibung

Die Erfindung liegt auf dem Gebiet der Kosmetik und betrifft ein Verfahren zur Herstellung eines konditionierenden Preshave- oder Aftershavemittels, bei dem eine Mikroemulsion mit einem einen speziellen Alk(en)yletherphosphat-Emulgator, Wasser und Öl enthaltenden kosmetischen Träger vermischt wird.

Die Erfindung betrifft weiterhin ein konditionierendes Preshave- oder Aftershavemittel, das eine Mikroemulsion, einen speziellen Alk(en)yletherphosphat-Emulgator, Wasser und Öl enthält, sowie die nicht-therapeutische Verwendung dieses konditionierenden Preshave- oder Aftershavemittels zur Verbesserung des Hautgefühls und/oder zur Linderung des Spannungsgefühls und des Brennens, das als Folge der Rasur auftritt.

Kosmetische Rasierhilfsmittel in Form von Cremes, Aerosolen, Gelen, Seifen oder Schäumen sind seit langem bekannt und werden stets weiter entwickelt und/oder den wachsenden Anforderungen der Verbraucher angepasst. Sie werden üblicherweise kurz vor der Rasur - als so genannte Preshavemittel - oder im Anschluss an eine Rasur - als so genannte Aftershavemittel - auf die Haut aufgetragen.

Klassische Rasierseifen weisen oftmals den Nachteil auf, dass sie sich nicht optimal auf der Haut verteilen lassen und die Haut reizen. Gele und/oder Cremes enthalten in der Regel spezielle Polymere und/oder Fettstoffe und weisen gegenüber klassischen Seifen eine bessere Verteilbarkeit auf der Haut und eine höhere Milde auf. Die zusätzlichen Pflegestoffe können jedoch auch zu einer allzu großen Schlüpfrigkeit der Haut führen, so dass die Rasierklinge über die Haut hinweg gleitet, ohne dass die Haare vollständig entfernt werden.

In der Anmeldung WO 95/05147 wurden u.a. Rasierschäume offenbart, die eine Polyorganosiloxan-Mikroemulsion enthalten und die vor und nach der Rasur ein gutes Hautgefühl hinterlassen.

US 2011/262370A1 offenbart Rasierzusammensetzungen, die ein Alkyloligoglycosid enthalten, aber nicht als Mikroemulsion vorliegen, sowie Verfahren zur Herstellung dieser Zusammensetzungen. WO 2009/051486A1 offenbart emulsionsförmige Rasierzusammensetzungen, nicht als Mikroemulsion vorliegen. EP 813862A2 offenbart Parfümölkonzentrate in Form wässriger Mikroemulsionen, enthaltend 1 - 30 Gew.-% Alkylglycoside und 1 - 10 Gew.-% einer Co-Ölkomponente, die bevorzugt ausgewählt ist aus Dialkylethern. EP 507047A2 offenbart Mikroemulsionen mit hoher Haut- und Schleimhautverträglichkeit, die Alkylpolyglycoside und etherische Öle sowie gegebenenfalls weitere Öle und Coemulgatoren enthalten. WO 2012/130655A2 offenbart ein Verfahren zur Herstellung eines konditionierenden Reinigungsmittels, bei dem eine Mikroemulsion, enthaltend mindestens ein Alkyl(oligo)glycosid, mindestens einen Mono- oder Diester von Glycerin mit mindestens einer C₁₀-C₂₄-Fettsäure, mindestens ein Öl und Wasser, mit einem kosmetischen Träger vermischt wird, der eine ausgewählte Polyhydroxyverbindung enthält.

Ein großes Problem bei der Rasur stellt die starke mechanische Reizung der Haut und das damit verbundene unangenehme Hautgefühl dar. Eine besonders beliebte Applikationsform für Rasierhilfsmittel für die Rasur gereizter, sensibler, empfindlicher, unreiner und/oder mit Akne belasteter Haut sind Rasierbalsame und Rasierlotionen, die einen mittleren Ölgehalt aufweisen. Für die genannten Hauttypen steht insbesondere die Verbesserung des Hautgefühls, insbesondere die Linderung des Spannungsgefühls und des Brennens, das als Folge der Rasur auftritt.

Aufgabe der vorliegenden Erfindung war es daher, Rasierhilfsmittel für die Rasur besonders empfindlicher Haut(partien) bereitzustellen, denn allein durch die Einarbeitung höherer Mengen an Weichmachern konnten Hautreizungen und/oder kleine Verletzungen der Haut, die durch die Rasierklinge bei der Nassrasur entstehen können, in der Vergangenheit nicht immer verhindert werden.

Die Rasierhilfsmittel sollten der behaarten Hautoberfläche eine ausreichende Geschmeidigkeit verleihen, um die Gleitfähigkeit der Rasierklinge zu erhöhen, ohne jedoch die Haut zu verletzen. Ein weiteres Ziel war die Herstellung eines Rasierhilfsmittels mit hohem Hautpflegeeffekt, das der Haut während und/oder nach der Rasur keine allzu hohe Schlüpfrigkeit verleiht.

Der vorliegenden Erfindung lag weiterhin die Aufgabe zugrunde, ein unkompliziertes Verfahren zur Herstellung eines konditionierenden Preshave- oder Aftershavemittels bereitzustellen.

Ein weiteres Ziel der Erfindung war es, transparente Preshave- oder Aftershavemittel herzustellen.

Es wurde gefunden, dass die Kombination mikroemulgierter Öle mit bestimmten Emulgatoren in Rasierhilfsmitteln zu besonders pflegenden Produkten führt, die sich in einfacher Weise herstellen lassen.

Gegenstand der Erfindung ist ein Verfahren zur Herstellung eines konditionierenden Preshave- oder Aftershavemittels, das die folgenden Schritte aufweist:
a) Bereitstellen einer Mikroemulsion, enthaltend
   a) i) mindestens ein Alkyl(oligo)glycosid,
   a) ii) mindestens einen Mono- oder Diester von Glycerin mit mindestens einer C₁₀-C₂₄-Fettsäure als Coemulgator, zusätzlich dazu
   a) iii) mindestens ein Öl und
   a) iv) Wasser,
   wobei die Komponenten (i), (ii), (iii) und (iv), jeweils bezogen auf das Gewicht der Mikroemulsion, in den Mengen I, II, III und IV vorliegen mit
   I = 15 - 25 Gew.-%, bezogen auf das Gewicht der Mikroemulsion,
   II = 4 - 10 Gew.-%, bezogen auf das Gewicht der Mikroemulsion,
   III = 15 - 25 Gew.-%, bezogen auf das Gewicht der Mikroemulsion,
   IV = 40 - 66 Gew.-%, bezogen auf das Gewicht der Mikroemulsion,
   und
b) Vermischen der Mikroemulsion mit einem Wasser-haltigen kosmetischen Träger, der enthält:
   b) i) als Emulgator mindestens ein Alkylether- oder Alkenyletherphosphat der Formel (I), 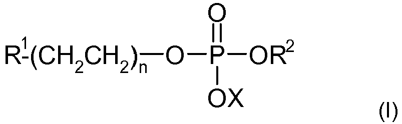 in der R¹ für einen aliphatischen Kohlenwasserstoffrest mit 8 bis 30 Kohlenstoffatomen, R² für Wasserstoff, einen Rest (CH₂CH₂O)ₙR¹ oder X, n für Zahlen von 1 bis 10 und X für Wasserstoff, einen Rest (CH₂CH₂O)ₙR¹, ein Alkali- oder Erdalkalimetall oder NR³R⁴R⁵R⁶, mit R³ bis R⁶ unabhängig voneinander stehend für einen C₁ bis C₄ - Kohlenwasserstoffrest, steht, und
   b) ii) mindestens ein -von a) iii) verschiedenes - Öl.

Mikroemulsionen a), die sich für die Verwendung in dem erfindungsgemäßen Verfahren eignen, sind bevorzugt thermodynamisch stabile Mischungen aus Öl, einem Emulgator und einem Coemulgator. Besonders bevorzugte Mikroemulsionen weisen eine Volumen-mittlere Partikelgröße im Bereich von 10 nm bis 150 nm, bevorzugt von 20 nm bis 100 nm und besonders bevorzugt von 30 bis 80 nm, auf.

Unter einem kosmetischen Träger wird ein wässriger oder wässrig-alkoholischer Träger verstanden.

Der kosmetische Wasser-haltige Träger enthält daneben als Emulgator mindestens ein Alkylether- oder Alkenyletherphosphat der Formel (I), 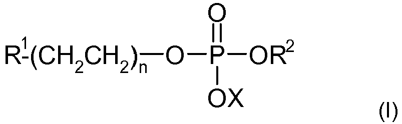 in der R¹ für einen aliphatischen Kohlenwasserstoffrest mit 8 bis 30 Kohlenstoffatomen, R² für Wasserstoff, einen Rest (CH₂CH₂O)ₙR¹ oder X, n für Zahlen von 1 bis 10 und X für Wasserstoff, einen Rest (CH₂CH₂O)ₙR¹, ein Alkali- oder Erdalkalimetall oder NR³R⁴R⁵R⁶, mit R³ bis R⁶ unabhängig voneinander stehend für einen C₁ bis C₄ - Kohlenwasserstoffrest, steht.

Ein bevorzugter Emulgator der Formel (I) ist dadurch gekennzeichnet, dass die Reste R¹ für mindestens eine Gruppe, ausgewählt Lauryl, Myristyl, Cetyl, Stearyl oder Oleyl sowie Mischungen hiervon, stehen; besonders bevorzugt sind Emulgatoren der Formel (I), in denen die Reste R¹ für die Mischung Cetearyl stehen.

Weitere bevorzugte Emulgatoren der Formel (I) sind dadurch gekennzeichnet, dass die Reste R² und X für einen Rest (CH₂CH₂O)ₙR¹ stehen, in denen die Reste R¹ für mindestens eine Gruppe, ausgewählt Lauryl, Myristyl, Cetyl, Stearyl oder Oleyl sowie Mischungen hiervon, stehen.

Weitere bevorzugte Emulgatoren der Formel (I) sind dadurch gekennzeichnet, dass n für Zahlen von 2 bis 6, bevorzugt 3 bis 5, besonders bevorzugt 4, steht.

Besonders bevorzugte Emulgatoren der Formel (I) sind dadurch gekennzeichnet, dass die Reste R¹ für mindestens eine Gruppe, ausgewählt Lauryl, Myristyl, Cetyl, Stearyl oder Oleyl sowie Mischungen hiervon, stehen, insbesondere für die Mischung Cetearyl, weiterhin die Reste R² und X für einen Rest (CH₂CH₂O)ₙR¹ stehen, wobei R¹ die vorstehende Bedeutung hat und n für Zahlen von 2 bis 6, bevorzugt 3 bis 5, besonders bevorzugt 4, steht.

Ein erfindungsgemäß besonders bevorzugtes Verfahren ist dadurch gekennzeichnet, dass der Emulgator der Formel (I) ausgewählt ist aus Trilaureth-4-phosphat und Triceteareth-4-phosphat sowie Mischungen hiervon. Außerordentlich bevorzugt ist ein erfindungsgemäßes Verfahren, bei dem der Emulgator der Formel (I) ausgewählt ist aus Triceteareth-4-phosphat.

Das erfindungsgemäße Verfahren erfordert keine bestimmte Reihenfolge bei der Vermischung der Komponenten a) und b). Es ist prinzipiell möglich, eine Mischung b) aus Wasser, dem mindestens einen Alkylether- oder Alkenyletherphosphat der Formel (I) als Emulgator und dem mindestens einem Öl zunächst als Träger vorzulegen, und anschließend die Mikroemulsion a) dazuzugeben. Ebenso ist es möglich, zu der Mikroemulsion a) eine Mischung b) aus Wasser, dem mindestens einen Alkylether- oder Alkenyletherphosphat der Formel (I) als Emulgator und mindestens einem Öl hinzuzufügen.

In einer bevorzugten Ausführungsform wird zunächst ein kosmetischer Träger in Form der zuvor beschriebenen Mischung b) aus Wasser, dem mindestens einen Alkylether- oder Alkenyletherphosphat der Formel (I) als Emulgator und und mindestens einem Öl vorgelegt. Anschließend werden alle fakultativen Komponenten des Preshave- oder Aftershavemittels, wie beispielsweise Parfum oder Konservierungsstoffe, in die Trägermischung eingearbeitet. Hierbei kann es bevorzugt sein, dass die Trägermischung b) bei einer Temperatur im Bereich von 50 bis 95°C hergestellt wird und die Mikroemulson a) bei einer Temperatur von 12 bis 45°C zugegeben, insbesondere bei einer niedrigen Scherrate von bevorzugt 5 bis 100 Umdrehungen pro Minute eingerührt wird.

Optional wird abschließend die Viskosität des Preshave- oder Aftershavemittels mit Hilfe eines Verdickungsmittels auf den gewünschten Bereich eingestellt.

Geeignete Alkylether- oder Alkenyletherphosphate gemäß Formel (I) werden in dem erfindungsgemäßen Verfahren bevorzugt in einer Gesamtmenge von 0,1 bis 4 Gew.-%, bevorzugt 0,5 bis 2 Gew.-%, weiter bevorzugt 0,8 bis 1,5 Gew.-%, jeweils bezogen auf das Gesamtgewicht des Preshave- oder Aftershavemittels, eingesetzt, wobei sich die Mengenangaben auf das Gesamtgewicht des konditionierenden Preshave- oder Aftershavemittels beziehen.

Mikroemulsionen a), die sich für die Verwendung in dem erfindungsgemäßen Verfahren eignen, enthalten - jeweils bezogen auf ihr Gesamtgewicht -
(i) 15 bis 25 Gew.-%, weiter bevorzugt 18 bis 22 Gew.-% mindestens eines Alkyl(oligo)-glycosids der allgemeinen Formel RO-[G]ₓ, in der R für einen Alkyl- und/oder Alkenylrest mit 8 bis 18 C-Atomen, bevorzugt mit 8 bis 14 C-Atomen, G für einen Zuckerrest mit 5 oder 6 C-Atomen, insbesondere für einen Glucose-Rest, und x für Zahlen von 1 bis 5, bevorzugt für Zahlen von 1 bis 3, steht,
(ii) 4 bis 10 Gew.-%, weiter bevorzugt 6 bis 9 Gew.-% und insbesondere 7 bis 8 Gew.-% mindestens eines Monoesters und/oder Diesters von Glycerin mit einer gesättigten oder ungesättigten, verzweigten oder unverzweigten C₁₀-C₂₄-Fettsäure,
(iii) 15 bis 25 Gew.-%, weiter bevorzugt 18 bis 22 Gew.-% mindestens eines Öls und
(iv) 40 bis 66 Gew.-% Wasser.

Besonders geeignete Alkyl(oligo)glycoside a(i) leiten sich von Aldosen und/oder Ketosen mit 5 oder 6 Kohlenstoffatomen, vorzugsweise von Glucose, ab.

Der Rest R steht besonders bevorzugt für einen Alkylrest mit 8 bis 14 und insbesondere mit 10 bis 12 Kohlenstoffatomen, insbesondere für n-Octyl, n-Decyl und Lauryl.

Die Indexzahl x steht in der allgemeinen Formel RO-[G]ₓ für den Oligomerisierungsgrad (DP), d.h. für die Verteilung der Mono- und Oligoglycoside. Die Indexzahl x weist vorzugsweise einen Wert im Bereich von 1 bis 5, besonders bevorzugt im Bereich von 1 bis 3 auf, wobei es sich dabei um keine ganze Zahl, sondern um eine gebrochene Zahl handeln kann, die analytisch ermittelt werden kann. Besonders bevorzugte Alkyl(oligo)glycoside haben einen Oligomerisierungsgrad von 1,2 bis 1,5. Besonders geeignete Alkyl(oligo)glycoside sind im Handel von verschiedenen Anbietern erhältlich unter den INCI-Bezeichnungen Decyl Glucoside, Lauryl Glucoside und Coco Glucoside. Besonders geeignete Ester a(ii) sind Monoester des Glycerins mit linearen Fettsäuren, die Alkylkettenlängen von 12 bis 22 C-Atomen aufweisen. Beispiele für besonders geeignete Ester (ii) sind Glycerylmonolaurat, Glycerylmonomyristat, Glycerylmonopalmitat, Glycerylmonostearat und/oder Glycerylmonooleat. Insbesondere geeignet ist Glycerylmonooleat.

Geeignete Öle a(iii) können ausgewählt sein aus mineralischen, natürlichen und synthetischen Ölkomponenten und/oder Fettstoffen.

Bevorzugte mikroemulgierte Ölkomponenten a(iii) sind ausgewählt aus Dialkylethern. Besonders bevorzugte Dialkylether sind Di-n-alkylether mit insgesamt 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-undecylether sowie Di-tert.-butylether, Di-iso-pentylether, Di-3-ethyldecylether, tert.-Butyl-n-octylether, iso-Pentyl-n-octylether und 2-Methylpentyl-n-octylether. Besonders bevorzugt ist der Di-n-octylether, der im Handel beispielsweise unter der Bezeichnung Cetiol® OE erhältlich ist.

Als natürliche Öle a(iii) können Triglyceride und Mischungen von Triglyceriden eingesetzt werden. Bevorzugte natürliche Öle sind Kokosnussöl, (süßes) Mandelöl, Walnussöl, Pfirsichkernöl, Aprikosenkernöl, Avocadoöl, Teebaumöl (Tea Tree Oil), Sojaöl, Sesamöl, Sonnenblumenöl, Tsubakiöl, Nachtkerzenöl, Reiskleieöl, Palmkernöl, Mangokernöl, Wiesenschaumkrautöl, Distelöl, Macadamianussöl, Traubenkernöl, Amaranthsamenöl, Arganöl, Bambusöl, Olivenöl, Weizenkeimöl, Kürbiskernöl, Malvenöl, Haselnussöl, Safloröl, Canolaöl, Sasanquaöl, Jojobaöl, Rambutanöl, Kakaobutter und Shea-Butter.

Als mineralische Öle a(iii) kommen insbesondere Mineralöle, Paraffin- und Isoparaffinöle sowie synthetische Kohlenwasserstoffe zum Einsatz. Ein Beispiel für einen einsetzbaren Kohlenwasserstoff ist beispielsweise das als Handelsprodukt erhältliche 1,3-Di-(2-ethylhexyl)-cyclohexan.

Als synthetische Öle kommen Silikonöle in Betracht.

Auch Fettalkohole, insbesondere verzweigte und/oder ungesättigte Fettalkohole, können als Ölkomponente a(iii) eingesetzt werden, beispielsweise Octenol, Dodecenol, Decenol, Octadienol, Dodecadienol, Decadienol, Oleylalkohol, Erucaalkohol, Ricinolalkohol, Isostearylalkohol, Linoleylalkohol und Linolenylalkohol sowie deren Guerbetalkohole.

Weitere mikroemulgierbare Öle a)iii) sind beispielsweise Esteröle. Unter Esterölen sind die Ester von C₆ - C₃₀ - Fettsäuren mit C₂ - C₃₀ - Fettalkoholen zu verstehen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen.

Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Besonders bevorzugt sind Isopropylmyristat, Isononansäure-C16-18-alkylester, 2-Ethylhexylpalmitat, Stearinsäure-2-ethylhexylester, Cetyloleat, Glycerintricaprylat, Kokosfettalkohol-caprinat/- caprylat, n-Butylstearat, Oleylerucat, Isopropylpalmitat, Oleyloleat, Laurinsäurehexylester, Di-n-butyladipat, Myristylmyristat, Cetearylisononanoat und Ölsäuredecylester sowie Mischungen dieser Ester.

Weitere mikroemulgierbare Öle a)iii) sind beispielsweise Dicarbonsäureester, wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-di-isostearat, Neopentylglykoldicaprylat.

Weitere mikroemulgierbare Öle a)iii) sind beispielsweise symmetrische, unsymmetrische oder zyklische Ester der Kohlensäure mit Fettalkoholen, insbesondere Glycerincarbonat oder Dicaprylylcarbonat.

Ein weiterer wesentlicher Inhaltsstoff der Mikroemulsionen a) ist Wasser. Die Mikroemulsion a) enthält - bezogen auf ihr Gesamtgewicht - 40 bis 66 Gew.-%, bevorzugt 45 bis 60 Gew.-% und insbesondere 50 bis 55 Gew.-% Wasser.

Erfindungsgemäß besonders bevorzugte Mikroemulsionen a) enthalten, jeweils bezogen auf ihr Gewicht,
a) i) 15 - 25 Gew.-% C₈-C₁₆-Alkylglucoside mit einem Oligomerisierungsgrad von 1,2 bis 1,5,
a) ii) 4 - 10 Gew.-% Mischung aus Glycerylmonooleat und Glyceryldioleat,
a) iii) 15 - 25 Gew.-% Di-n-octylether und
a) iv) 40 - 66 Gew.-% Wasser.

Die Mikroemulsion a) kann (vor der Durchführung des erfindungsgemäßen Verfahrens) bevorzugt durch Vermischen der flüssigen Ölphasen (ii) und (iii) mit der tensidhaltigen, wässrigen Phase ((i) und (iv)) unter Rühren hergestellt werden.

Alternativ kann die Mikroemulsion a) in dem erfindungsgemäßen Verfahren auch als vorgefertigtes Handelsprodukt eingesetzt werden. Ein Beispiel für eine erfindungsgemäß bevorzugte, im Handel erhältliche Mikroemulsion a) ist die unter der Bezeichnung "Plantasil Micro®" von der Firma Cognis erhältliche Mikroemulsion.

Die Mikroemulsion a) wird in dem erfindungsgemäßen Verfahren bevorzugt in einer Menge von 0,01 bis 50 Gew.-%, weiter bevorzugt von 0,1 bis 30 Gew.-%, besonders bevorzugt von 0,5 bis 20 Gew.-% und insbesondere von 1 bis 15 Gew.-% eingesetzt, wobei sich die Mengen auf das Gesamtgewicht des konditionierenden Preshave- oder Aftershavemittels beziehen.

Als weitere obligatorische Komponente ist in der Wasser-haltigen Trägermischung b) des konditionierenden Preshave- oder Aftershavemittels mindestens ein Öl b)ii) enthalten, das von dem mikroemulgierten Öl a) iii) verschieden ist.

Durch die Auswahl verschiedener Öle erhält man eine Optimierung der Anwendungseigenschaften, insbesondere im Hinblick auf die Verteilbarkeit des finalen Produkts auf der Haut und das dabei erzielte Hautgefühl. Prinzipiell sind als Öle b)ii aber auch die zur Mikroemulgierung geeigneten Öle a)iii) geeignet, die aus mineralischen, natürlichen und synthetischen Ölkomponenten und/oder Fettstoffen ausgewählt sein können.

Bevorzugte Ölkomponenten b) ii) sind ausgewählt aus Esterölen. Unter Esterölen sind die Ester von C₆ - C₃₀ - Fettsäuren mit C₂ - C₃₀ - Fettalkoholen zu verstehen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen.

Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Besonders bevorzugt sind Isopropylmyristat, Isononansäure-C16-18-alkylester, 2-Ethylhexylpalmitat, Stearinsäure-2-ethylhexylester, Cetyloleat, Glycerintricaprylat, Kokosfettalkohol-caprinat/- caprylat, n-Butylstearat, Oleylerucat, Isopropylpalmitat, Oleyloleat, Laurinsäurehexylester, Di-n-butyladipat, Myristylmyristat, Cetearylisononanoat und Ölsäuredecylester sowie Mischungen dieser Ester. Besonders bevorzugte Esteröle sind die Ester von C₁₂ - C₂₄ - Fettsäuren mit C₁₂ - C₂₄ - Fettalkoholen. Diese Esteröle spreiten langsamer als die Esteröle mit weniger Kohlenstoffatomen im Molekül und stellen für die Anwendungseigenschaften des Endprodukts eine gute Ergänzung zu den mikroemulgierten Ölen mit geringerem Molekulargewicht dar.

Weitere geeignete Öle b)ii) sind Dicarbonsäureester, wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-di-isostearat, Neopentylglykoldicaprylat.

Weitere geeignete Öle b)ii) sind symmetrische, unsymmetrische oder cyclische Ester der Kohlensäure mit Fettalkoholen, insbesondere Glycerincarbonat oder Dicaprylylcarbonat.

Weitere besonders geeignete Öle b)ii) sind ausgewählt aus Dialkylethern. Besonders bevorzugte Dialkylether sind Di-n-alkylether mit insgesamt 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyln-dodecylether und n-Hexyl-n-undecylether sowie Di-tert.-butylether, Di-iso-pentylether, Di-3-ethyldecylether, tert.-Butyl-n-octylether, iso-Pentyl-n-octylether und 2-Methylpentyl-n-octylether. Besonders bevorzugt ist der Di-n-octylether, der im Handel beispielsweise unter der Bezeichnung Cetiol® OE erhältlich ist.

Als natürliche Öle b)ii) können Triglyceride und Mischungen von Triglyceriden eingesetzt werden. Bevorzugte natürliche Öle sind Kokosnussöl, (süßes) Mandelöl, Walnussöl, Pfirsichkernöl, Aprikosenkernöl, Avocadoöl, Teebaumöl (Tea Tree Oil), Sojaöl, Sesamöl, Sonnenblumenöl, Tsubakiöl, Nachtkerzenöl, Reiskleieöl, Palmkernöl, Mangokernöl, Wiesenschaumkrautöl, Distelöl, Macadamianussöl, Traubenkernöl, Amaranthsamenöl, Arganöl, Bambusöl, Olivenöl, Weizenkeimöl, Kürbiskernöl, Malvenöl, Haselnussöl, Safloröl, Canolaöl, Sasanquaöl, Jojobaöl, Rambutanöl, Kakaobutter und Shea-Butter.

Als mineralische Öle b)ii) kommen insbesondere Mineralöle, Paraffin- und Isoparaffinöle sowie synthetische Kohlenwasserstoffe zum Einsatz. Ein Beispiel für einen einsetzbaren Kohlenwasserstoff ist beispielsweise das als Handelsprodukt erhältliche 1,3-Di-(2-ethylhexyl)-cyclohexan.

Als synthetische Öle b)ii) kommen Silikonöle in Betracht.

Auch Fettalkohole, insbesondere verzweigte und/oder ungesättigte Fettalkohole, können als Ölkomponente b)ii) eingesetzt werden, beispielsweise Octenol, Dodecenol, Decenol, Octadienol, Dodecadienol, Decadienol, Oleylalkohol, Erucaalkohol, Ricinolalkohol, Isostearylalkohol, Linoleylalkohol und Linolenylalkohol sowie deren Guerbetalkohole.

In einer besonders bevorzugten Ausführungsform kann die Trägermischung b) zusätzlich zu dem mindestens einen Öl b)ii), das von dem mindestens einen mikroemulgierten Öl a)iii) verschieden ist, auch das Öl a)iii) in nicht mikroemulgierter Form enthalten.

In einer weiteren besonders bevorzugten Ausführungsform enthält das erfindungsgemäß hergestellte oder erfindungsgemäße Preshave- oder Aftershavemittel, jeweils bezogen auf sein Gesamtgewicht, insgesamt 1 - 7 Gew.-% Öle, bevorzugt 2 - 6 Gew.-% Öle, besonders bevorzugt 3 - 5 Gew.-% Öle, einschließlich aller mikroemulgierten und nicht mikroemulgierten Öle.

In einer weiteren besonders bevorzugten Ausführungsform enthält das erfindungsgemäß hergestellte oder erfindungsgemäße Preshave- oder Aftershavemittel, jeweils bezogen auf sein Gesamtgewicht, insgesamt 0,05 - 5 Gew.-%, bevorzugt 0,1 - 3 Gew.-%, besonders bevorzugt 0,2 - 1,5 Gew.-% eines oder mehrerer Öle in mikroemulgierter Form.

In einer weiteren besonders bevorzugten Ausführungsform enthält das erfindungsgemäß hergestellte oder erfindungsgemäße Preshave- oder Aftershavemittel, jeweils bezogen auf sein Gesamtgewicht, insgesamt 0,95 - 6,95 Gew.-%, bevorzugt 2 - 5 Gew.-%, besonders bevorzugt 3 - 4 Gew.-%, eines oder mehrerer Öle in nicht mikroemulgierter Form.

In einer weiteren besonders bevorzugten Ausführungsform enthält das erfindungsgemäß hergestellte oder erfindungsgemäße Preshave- oder Aftershavemittel, jeweils bezogen auf sein Gesamtgewicht, insgesamt 1 - 7 Gew.-% Öle, bevorzugt 2 - 6 Gew.-% Öle, besonders bevorzugt 3 - 5 Gew.-% Öle, davon 0,05 - 5 Gew.-%, bevorzugt 0,1 - 3 Gew.-%, besonders bevorzugt 0,2 - 1,5 Gew.-% eines oder mehrerer Öle in mikroemulgierter Form und 0,95 - 6,95 Gew.-%, bevorzugt 2 - 5 Gew.-%, besonders bevorzugt 3 - 4 Gew.-%, eines oder mehrerer Öle in nicht mikroemulgierter Form.

In einer besonders bevorzugten Ausführungsform führt das erfindungsgemäße Herstellungsverfahren zu konditionierenden Preshave- oder Aftershavemitteln, die transparent sind.

Unter "transparent" wird verstanden, dass die konditionierenden Preshave- oder Aftershavemittel einen NTU-Wert (nephelometric turbidity unit) bei 20°C von maximal 100, bevorzugt von maximal 50 und insbesondere von maximal 30 aufweisen.

Um die Stabilität und die besonderen Pflegeeigenschaften der Mikroemulsion a) nach dem Vermischen mit der Trägermischung b) zu bewahren, kann es bevorzugt sein, den Tensidgehalt der Trägermischung b), abgesehen von dem mindestens einen Alkylether- oder Alkenyletherphosphat der Formel (I) als Emulgator b) i) den Zusatz weiterer Tenside hinsichtlich Menge und Art der Tenside zu beschränken.

In einer bevorzugten Ausführungsform enthält die Mikroemulsion a) außer den 15 - 25 Gew.-% an Alkyl(oligo)glycosid(en) keine weiteren Tenside.

In einer weiteren bevorzugten Ausführungsform enthält das konditionierende Preshave- oder Aftershavemittel zusätzlich zu dem mindestens einen Alkylether- oder Alkenyletherphosphat der Formel (I) als Emulgator b) i) und den Tensiden aus der Mikroemulsion a) weitere Tenside in einer Gesamtmenge von 0 bis 1,5 Gew.-%, bevorzugt 0,01 bis 1 Gew.-%, weiter bevorzugt 0,1 bis 0,3 Gew.-%, jeweils bezogen auf das Gesamtgewicht des Preshave- oder Aftershavemittels.

Zu den optionalen anionischen Tensiden zählen:
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare oder verzweigte, gesättigte oder ungesättigte Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und/oder -dialkylester mit 8 bis 24 C-Atomen in der Alkylgruppe und Sulfobernsteinsäuremonoalkylpolyoxyethylester mit 8 bis 24 C-Atomen in der Alkylgruppe und 1 bis 6 Oxyethylgruppen,
- Alpha-Olefinsulfonate mit 8 bis 24 C-Atomen,
- Alkylsulfat- und/oder Alkylpolyglykolethersulfatsalze der Formel R-O(CH₂-CH₂O)ₓ-OSO₃⁻ X⁺, in der R eine bevorzugt lineare oder verzweigte, gesättigte oder ungesättigte Alkylgruppe mit 8 bis 30 C-Atomen, x = 0 oder 1 bis 12 und X ein Alkali- oder Ammoniumion ist,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Optionale amphotere/zwitterionische Tenside können ausgewählt sein aus Verbindungen der folgenden Formeln (i) bis (v), in denen der Rest R jeweils für einen geradkettigen oder verzweigten, gesättigten oder ein- bzw. mehrfach ungesättigten Alkyl- oder Alkenlyrest mit 8 bis 24 Kohlenstoffatomen steht,
(i) 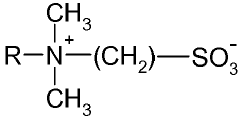
(ii) 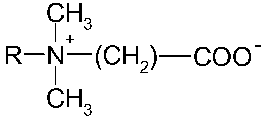
(iii) 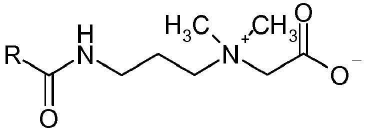
(iv) 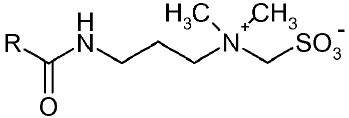
(v)

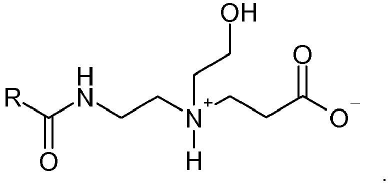

Besonders geeignete optionale amphotere/zwitterionische Tenside sind Alkylamidoalkylbetaine und/oder Alkylampho(di)acetate der zuvor genannten Formeln (i) bis (v), insbesondere die unter den INCI-Bezeichnungen Cocamidopropylbetain und Disodium Cocoamphodiacetate bekannten Tenside.

Optionale nichtionische Tenside/Emulgatoren sind beispielsweise
- C₈-C₃₀-Fettsäuremono- und -diester von Anlagerungsprodukten von 1 bis 30 Mol Ethylenoxid an Glycerin, beispielsweise das unter der INCI-Bezeichnung bekannte PEG-7 Glyceryl Cocoate
- Aminoxide,
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- Sorbitanfettsäureester und Anlagerungsprodukte von Ethylenoxid an Sorbitanfettsäureester wie beispielsweise Polysorbate,
- Zuckerfettsäureester und Anlagerungsprodukte von Ethylenoxid an Zuckerfettsäureester, und/oder
- Anlagerungsprodukte von Ethylenoxid an Fettsäurealkanolamide und Fettamine.

Die Pflegewirkung der durch das erfindungsgemäße Verfahren hergestellten konditionierenden Preshave- oder Aftershavemittel wird dadurch erzielt, dass die Lipidkomponente(n) aus der Mikroemulsion auf der Haut homogen verteilt und abgeschieden wird (werden). Überraschenderweise werden dadurch das Brennen und das Irritationsgefühl der durch die Rasur gereizten Haut spürbar gelindert. Bei der Verwendung als Preshavemittel wird zusätzlich das Gleiten der Rasierklinge auf der Haut erleichtert, ohne dass aber die Haut zu schlüpfrig wird und das Rasurergebnis dadurch beeinträchtigt würde.

Die obligatorischen Bestandteile der Trägermischung b), nämlich das mindestens ein Alkylether- oder Alkenyletherphosphat der Formel (I) als Emulgator b) i) und das mindestens eine - vom mikroemulgierten Öl a) iii) verschiedene - Öl tragen zur Haut beruhigenden und pflegenden Wirkung bei.

Das erfindungsgemäße konditionierende Preshave- oder Aftershavemittel kann noch eine Reihe weiterer fakultativer Wirkstoffe enthalten, die auf den Haaren vorteilhafte Eigenschaften bewirken können und das erfindungsgemäße Verfahren nicht erschweren. Zu den bevorzugten fakultativen Wirkstoffen zählen zum Beispiel:
Verdickungsmittel.

Besonders bevorzugte Verdickungsmittel für das erfindungsgemäß hergestellte bzw. erfindungsgemäße konditionierende Preshave- oder Aftershavemittel sind ausgewählt aus mindestens einem Homo- oder Copolymer von Acrylsäure, 2-Methyl-2[(1-oxo-2-propenyl)amino]-1-propansulfonsäure (AMPS), Acrylsäureamiden oder Acrylsäureestern sowie aus Mischungen der genannten Monomere. Besonders bevorzugte Herstellverfahren und Mittel sind dadurch gekennzeichnet, dass das mindestens eine Homo- oder Copolymer von Acrylsäure, 2-Methyl-2[(1-oxo-2-propenyl)amino]-1-propansulfonsäure, Acrylsäureamiden oder Acrylsäureestern b) ii) ausgewählt ist aus Polyacrylsäure, den Salzen von Polyacrylsäure, Homopolymeren der 2-Methyl-2[(1-oxo-2-propenyl)amino]-1-propansulfonsäure, Copolymeren von 2-Methyl-2[(1-oxo-2-propenyl)amino]-1-propansulfonsäure und Acrylamid, Copolymeren von 2-Methyl-2[(1-oxo-2-propenyl)amino]-1-propansulfonsäure und Acrylsäure, Copolymeren von 2-Methyl-2[(1-oxo-2-propenyl)amino]-1-propansulfonsäure und 2-Hydroxyethylacrylat, Copolymeren von 2-Methyl-2[(1-oxo-2-propenyl)amino]-1-propansulfonsäure und Polyvinylpyrrolidon, sowie Mischungen hiervon. Weitere besonders bevorzugte Herstellverfahren und Mittel sind dadurch gekennzeichnet, dass das erfindungsgemäß hergestellte bzw. erfindungsgemäße konditionierende Preshave- oder Aftershavemittel mindestens ein Verdickungsmittel, ausgewählt aus mindestens einem Homo- oder Copolymer von Acrylsäure, 2-Methyl-2[(1-oxo-2-propenyl)amino]-1-propansulfonsäure, Acrylsäureamiden oder Acrylsäureestern sowie aus Mischungen der genannten Monomere, in einer Gesamtmenge von 0,01 bis 1,5 Gew.-%, bevorzugt 0,1 bis 1,1 Gew.-%, besonders bevorzugt 0,3 bis 0,8 Gew.-%, jeweils bezogen auf das Gewicht des konditionierende Preshave- oder Aftershavemittels, enthält. Bevorzugte konditionierende Preshave- oder Aftershavemittel weisen eine Viskosität im Bereich von 2000 bis 30000 mPas, bevorzugt von 5000 bis 15000 mPas, auf, gemessen bei 20°C mit einem Rotationsviskosimeter.
- Vitamine, Vitaminderivate und/oder Vitaminvorstufen, die in dem erfindungsgemäßen Verfahren bevorzugt in einer Menge von 0,001 bis 3 Gew.-%, weiter bevorzugt von 0,005 bis 2 Gew.-% und insbesondere von 0,01 bis 1 Gew.-% eingesetzt werden können, wobei sich die Mengenangaben jeweils auf das Gesamtgewicht des konditionierenden Preshave- oder Aftershavemittels beziehen.

Unter geeigneten Vitaminen sind bevorzugt die folgenden Vitamine, Provitamine und Vitaminvorstufen sowie deren Derivate zu verstehen:
Vitamin A: zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen beispielsweise Vitamin A-Säure und deren Ester, Vitamin A-Aldehyd und Vitamin A-Alkohol sowie dessen Ester wie das Palmitat und das Acetat in Betracht.
Vitamin B: zur Vitamin B-Gruppe oder zu dem Vitamin B-Komplex gehören u. a.
   - Vitamin B₁ (Thiamin)
   - Vitamin B₂ (Riboflavin)
   - Vitamin B₃. Unter dieser Bezeichnung werden häufig die Verbindungen Nicotinsäure und Nicotinsäureamid (Niacinamid) geführt.
   - Vitamin B₅ (Pantothensäure und Panthenol). Im Rahmen dieser Gruppe wird bevorzugt das Panthenol eingesetzt. Einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie kationische Panthenolderivate.
   - Vitamin B₆ (Pyridoxin sowie Pyridoxamin und Pyridoxal).
Vitamin C (Ascorbinsäure): die Verwendung in Form des Palmitinsäureesters, der Glucoside oder Phosphate kann bevorzugt sein. Die Verwendung in Kombination mit Tocopherolen kann ebenfalls bevorzugt sein.
Vitamin E (Tocopherole, insbesondere α-Tocopherol).
Vitamin F: unter dem Begriff "Vitamin F" werden üblicherweise essentielle Fettsäuren, insbesondere Linolsäure, Linolensäure und Arachidonsäure, verstanden.
Vitamin H: Als Vitamin H wird die Verbindung (3aS,4S, 6aR)-2-Oxohexahydrothienol[3,4-d]-imidazol-4-valeriansäure bezeichnet, für die sich der Trivialname Biotin durchgesetzt hat.

Weitere Wirk-, Hilfs- und Zusatzstoffe, die in dem erfindungsgemäßen Verfahren eingesetzt werden können, sind beispielsweise Pflanzenextrakte, Feuchthaltemittel, Parfüms, UV-Filter, Farbstoffe zum Anfärben des Mittels, Substanzen zur Einstellung des pH-Wertes, beispielsweise alpha- und beta-Hydroxycarbonsäuren wie Citronensäure, Milchsäure, Äpfelsäure, Glycolsäure, Wirkstoffe wie Bisabolol oder Allantoin, Komplexbildner wie EDTA, NTA, β-Alanindiessigsäure und Phosphonsäuren, Antioxidantien und Konservierungsmittel, wie beispielsweise Natriumbenzoat oder Salicylsäure.

Das erfindungsgemäße Verfahren eignet sich bevorzugt für die Herstellung konditionierender Preshave- oder Aftershavemittel, die einen pH-Wert im Bereich von 4,0 bis 7,5, bevorzugt von 4,5 bis 6,5 und insbesondere von 4,5 bis 5,0 aufweisen.

Das erfindungsgemäße Verfahren weist den Vorteil auf, dass es besonders einfach durchzuführen ist und einen geringen Energieaufwand erfordert. Die Pflegekomponenten a) und b) lassen sich in beliebiger Reihenfolge miteinander vermischen und es können weitere Hilfs- und Wirkstoffe in den kosmetischen Träger eingearbeitet werden, ohne dass das Verfahren dadurch merklich komplizierter wird.

Ferner lassen sich mit dem erfindungsgemäßen Verfahren konditionierende Preshave- oder Aftershavemittel herstellen, die transparent und stabil sind.

Die Hautbehandlung mit einem erfindungsgemäßen konditionierenden Preshave- oder Aftershavemittel, das bevorzugt über das erfindungsgemäße oder ein erfindungsgemäß bevorzugtes Verfahren zugänglich ist, bewirkt eine Verbesserung des Hautgefühls und/oder eine Linderung des Spannungsgefühls und des Brennens, das als Folge der Rasur auftritt.

Ein zweiter Gegenstand der Erfindung ist ein konditionierendes Preshave- oder Aftershavemittel, enthaltend
a) eine Mikroemulsion, enthaltend
   a) i) mindestens ein Alkyl(oligo)glycosid,
   a) ii) mindestens einen Mono- oder Diester von Glycerin mit mindestens einer C₁₀-C₂₄-Fettsäure als Coemulgator,
   a) iii) mindestens ein Öl und
   a) iv) Wasser,
   wobei die Komponenten (i), (ii), (iii) und (iv), jeweils bezogen auf das Gewicht der Mikroemulsion, in den Mengen I, II, III und IV vorliegen mit
   I = 15 - 25 Gew.-%, bezogen auf das Gewicht der Mikroemulsion,
   II = 4 - 10 Gew.-%, bezogen auf das Gewicht der Mikroemulsion,
   III = 15 - 25 Gew.-%, bezogen auf das Gewicht der Mikroemulsion,
   IV = 40 - 66 Gew.-%, bezogen auf das Gewicht der Mikroemulsion,
   weiterhin
   mindestens einen Emulgator der Formel (I) in einer Gesamtmenge von 0,1 bis 4 Gew.-%, bezogen auf das Gesamtgewicht des Preshave- oder Aftershavemittels, 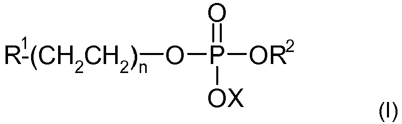 in der R¹ für einen aliphatischen Kohlenwasserstoffrest mit 8 bis 30 Kohlenstoffatomen, R² für Wasserstoff, einen Rest (CH₂CH₂O)ₙR¹ oder X, n für Zahlen von 1 bis 10 und X für Wasserstoff, einen Rest (CH₂CH₂O)ₙR¹, ein Alkali- oder Erdalkalimetall oder NR³R⁴R⁵R⁶, mit R³ bis R⁶ unabhängig voneinander stehend für einen C₁ bis C₄ - Kohlenwasserstoffrest, steht, und
b) ii) mindestens ein Öl, das von a)iii) verschieden ist,
   wobei das Preshave- oder Aftershavemittel - jeweils bezogen auf sein Gesamtgewicht - insgesamt 50 - 95 Gew.-% Wasser und insgesamt 1 - 7 Gew.-% Öl(e) enthält.

Ein dritter Gegenstand der Erfindung ist die Verwendung des zuvor beschriebenen kosmetischen Preshave- oder Aftershavemittels zur Verbesserung des Hautgefühls und/oder zur Linderung des Spannungsgefühls und des Brennens, das als Folge der Rasur auftritt.

Bezüglich weiterer bevorzugter Ausführungsformen des erfindungsgemäßen Mittels sowie der erfindungsgemäßen Verwendung gilt mutatis mutandis das zu dem erfindungsgemäßen Verfahren Gesagte.

### Beispiele:

**Konditionierendes Aftershave (alle Mengenangaben in Gew.-%. bezogen auf das Aftershavemittel)**

| | |
|---|---|
| Triceteareth-4 Phosphat | 1,0 |
| Propylparaben | 0,2 |
| Di-n-octylether (nicht mikroemulgiert) | 1 |
| Erucasäureoleylester (Oleyl Erucate, nicht mikroemulgiert)) | 2 |
| Hexandiol-1,6 | 6 |
| Methylparaben | 0,2 |
| Glycerin 99.5 Gew.-% | 3 |
| Wasser (Trägermischung b) | 80,94 |
| Milchsäure 80 Gew.-% DAB | 0,06 |
| Parfum | 0,6 |
| Mikroemulsion, darin | 3 |
| Wasser (ex Mikroemulsion) | 1,6 |
| Di-n-octylether (mikroemulgiert) | 0,6 |
| Glycerylmono-/-dioleat (ex Mikroemulsion) | 0,2 |
| C8-16-Alkylpolyglucoside (ex Mikroemulsion, INCI: Decyl Glucoside) | 0,6 |
| Sepigel 305 (von Seppic) | 2,0 |
| Wasser (ex Sepigel 305) | 0,7 |
| AMPS-Polyacrylamid-Copolymer (ex Sepigel 305) | 0,8 |
| Laureth-7 (ex Sepigel 305) | 0,1 |
| C13-14 Isoparaffin (ex Sepigel 305) | 0,4 |

### Herstellung:

Aus den in der Tabelle angegebenen Bestandteilen Wasser, Di-n-octylether, Glycerylmono-/-dioleat und C8-16-Alkylpolyglucoside (INCI: Decyl Glucoside) in den angegebenen Mengen wurde durch einfaches Vermischen bei einer Temperatur im Bereich von 15 bis 35°C eine Mikroemulsion hergestellt.

Getrennt davon wurden Triceteareth-4 Phosphat, Propylparaben, Di-n-octylether (nicht mikroemulgiert) und Erucasäureoleylester (nicht mikroemulgiert) gemeinsam aufgeschmolzen, vermischt und auf 90°C erhitzt (→ Ölphase).
Das Wasser (Trägermischung b) wurde mit Glycerin, Hexandiol-1,6 und Methylparaben vermischt und auf 90°C erhitzt (→ Wasserphase).
Die heiße Ölphase und die heiße Wasserphase wurden miteinander vermischt und zu einer Emulsion homogenisiert, die die Trägermischung b darstellt.
Diese Trägermischung b wurde unter Rühren auf 40°C abgekühlt. Dann wurden die Mikroemulsion, das Parfüm und die Milchsäure zugegeben.
Abschließend wurde die Verdickungsmittelzubereitung (Sepigel 305) zugegeben.

Das so hergestellte konditionierende Aftershavemittel wies einen pH-Wert von ca. 4,6 auf, gemessen bei 20 °C.
Die Viskosität betrug etwa 6000 mPas, gemessen bei 20°C mit einem Rotationsviskosimeter.

Das konditionierende Aftershavemittel wurde direkt nach der Rasur auf die rasierte Gesichtshaut aufgetragen. Alle Probanden verspürten nach der Rasur, aber vor dem Applizieren des Aftershavemittels ein Spannungsgefühl und Brennen der Haut. Durch das Auftragen des erfindungsgemäßen konditionierenden Aftershavemittels stellte sich eine sofortige Linderung des Spannungsgefühls und des Brennens ein. Weiterhin gaben die Probanden an, dass sie 30 Minuten nach der Applikation des Aftershavemittels ein verbessertes, weiches, nicht spannendes Hautgefühl verspürten.

## Patentansprüche

1. Verfahren zur Herstellung eines konditionierenden Preshave- oder Aftershavemittels, das die folgenden Schritte aufweist:
a) Bereitstellen einer Mikroemulsion, enthaltend
a) i) mindestens ein Alkyl(oligo)glycosid,
a) ii) mindestens einen Mono- oder Diester von Glycerin mit mindestens einer C₁₀-C₂₄-Fettsäure als Coemulgator, zusätzlich dazu
a) iii) mindestens ein Öl und
a) iv) Wasser,
wobei die Komponenten (i), (ii), (iii) und (iv), jeweils bezogen auf das Gewicht der Mikroemulsion, in den Mengen I, II, III und IV vorliegen mit
I = 15 - 25 Gew.-%, bezogen auf das Gewicht der Mikroemulsion,
II = 4 - 10 Gew.-%, bezogen auf das Gewicht der Mikroemulsion,
III = 15 - 25 Gew.-%, bezogen auf das Gewicht der Mikroemulsion,
IV = 40 - 66 Gew.-%, bezogen auf das Gewicht der Mikroemulsion,
und
b) Vermischen der Mikroemulsion mit einem Wasser-haltigen kosmetischen Träger, der enthält:
b) i) als Emulgator mindestens ein Alkylether- oder Alkenyletherphosphat der Formel (I), 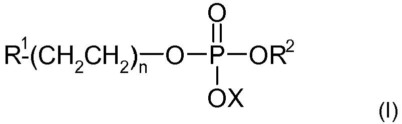 in der R¹ für einen aliphatischen Kohlenwasserstoffrest mit 8 bis 30 Kohlenstoffatomen, R² für Wasserstoff, einen Rest (CH₂CH₂O)ₙR¹ oder X, n für Zahlen von 1 bis 10 und X für Wasserstoff, einen Rest (CH₂CH₂O)ₙR¹, ein Alkali- oder Erdalkalimetall oder NR³R⁴R⁵R⁶, mit R³ bis R⁶ unabhängig voneinander stehend für einen C₁ bis C₄ - Kohlenwasserstoffrest, steht,
b) ii) mindestens ein -von a) iii) verschiedenes - Öl.

2. Verfahren nach Anspruch 1, **dadurch gekennzeichnet, dass** in Formel (I)
- die Reste R¹ für mindestens eine Gruppe, ausgewählt Lauryl, Myristyl, Cetyl, Stearyl oder Oleyl sowie Mischungen hiervon, stehen, insbesondere für die Mischung Cetearyl,
- die Reste R² und X für einen Rest (CH₂CH₂O)ₙR¹ stehen und
- n für Zahlen von 2 bis 6, bevorzugt 3 bis 5, besonders bevorzugt 4, steht.

3. Verfahren nach einem der Ansprüche 1 oder 2, **dadurch gekennzeichnet, dass** der Emulgator der Formel (I) ausgewählt ist aus Trilaureth-4-phosphat und Triceteareth-4-phosphat sowie Mischungen hiervon.

4. Verfahren nach einem der Ansprüche 1 bis 3, **dadurch gekennzeichnet, dass** das konditionierende Preshave- oder Aftershavemittel den mindestens einen Emulgator der Formel (I) in einer Gesamtmenge von 0,1 bis 4 Gew.-%, bevorzugt 0,5 bis 2 Gew.-%, weiter bevorzugt 0,8 bis 1,5 Gew.-%, jeweils bezogen auf das Gesamtgewicht des Preshave- oder Aftershavemittels, enthält.

5. Verfahren nach einem der Ansprüche 1 bis 4, **dadurch gekennzeichnet, dass** die Mikroemulsion a)
(i) insgesamt 15 bis 25 Gew.-% mindestens eines Alkyl(oligo)glycosids der allgemeinen Formel RO-[G]ₓ, in der R für einen Alkyl- und/oder Alkenylrest mit 4 bis 22 C-Atomen, G für einen Zuckerrest mit 5 oder 6 C-Atomen und x für Zahlen von 1 bis 10 steht,
(ii) insgesamt 4 bis 10 Gew.-% mindestens eines Monoesters und/oder Diesters von Glycerin mit einer gesättigten oder ungesättigten, verzweigten oder unverzweigten C₁₀-C₂₄-Fettsäure,
(iii) insgesamt 15 bis 25 Gew.-% mindestens eines von a)ii) verschiedenen Öls und
(iv) 40 bis 66 Gew.-% Wasser enthält,
wobei sich die Mengenangaben auf das Gewicht der Mikroemulsion a) beziehen.

6. Verfahren nach einem der Ansprüche 1 bis 5, **dadurch gekennzeichnet, dass** das konditionierende Preshave- oder Aftershavemittel - bezogen auf sein Gesamtgewicht - 0,5 bis 50 Gew.-%, bevorzugt 1,0 bis 30 Gew.-%, weiter bevorzugt 5,0 bis 10 Gew.-% der Mikroemulsion a) enthält.

7. Konditionierendes Preshave- oder Aftershavemittel, enthaltend
a) eine Mikroemulsion, enthaltend
a) i) mindestens ein Alkyl(oligo)glycosid,
a) ii) mindestens einen Mono- oder Diester von Glycerin mit mindestens einer C₁₀-C₂₄-Fettsäure als Coemulgator,
a) iii) mindestens ein Öl und
a) iv) Wasser,
wobei die Komponenten (i), (ii), (iii) und (iv), jeweils bezogen auf das Gewicht der Mikroemulsion, in den Mengen I, II, III und IV vorliegen mit
I = 15 - 25 Gew.-%, bezogen auf das Gewicht der Mikroemulsion,
II = 4 - 10 Gew.-%, bezogen auf das Gewicht der Mikroemulsion,
III = 15 - 25 Gew.-%, bezogen auf das Gewicht der Mikroemulsion,
IV = 40 - 66 Gew.-%, bezogen auf das Gewicht der Mikroemulsion,
weiterhin
mindestens einen Emulgator der Formel (I) in einer Gesamtmenge von 0,1 bis 4 Gew.-%, bezogen auf das Gesamtgewicht des Preshave- oder Aftershavemittels, 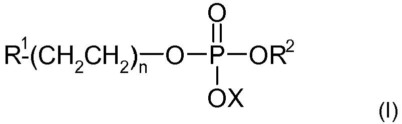 in der R¹ für einen aliphatischen Kohlenwasserstoffrest mit 8 bis 30 Kohlenstoffatomen, R² für Wasserstoff, einen Rest (CH₂CH₂O)ₙR¹ oder X, n für Zahlen von 1 bis 10 und X für Wasserstoff, einen Rest (CH₂CH₂O)ₙR¹, ein Alkali- oder Erdalkalimetall oder NR³R⁴R⁵R⁶, mit R³ bis R⁶ unabhängig voneinander stehend für einen C₁ bis C₄ - Kohlenwasserstoffrest, steht, und
b) ii) mindestens ein Öl, das von a)iii) verschieden ist,
wobei das Preshave- oder Aftershavemittel - jeweils bezogen auf sein Gesamtgewicht - insgesamt 50 - 95 Gew.-% Wasser und insgesamt 1 - 7 Gew.-% Öl(e) enthält.

8. Konditionierendes Preshave- oder Aftershavemittel nach Anspruch 7 oder Verfahren nach einem der Ansprüche 1 bis 6, **dadurch gekennzeichnet, dass** die Mikroemulsion a), jeweils bezogen auf ihr Gewicht,
a) i) 15 - 25 Gew.-% C₈-C₁₆-Alkylglucoside mit einem Oligomerisierungsgrad von 1,2 bis 1,5,
a) ii) 4 - 10 Gew.-% Mischung aus Glycerylmonooleat und Glyceryldioleat,
a) iii) 15 - 25 Gew.-% Di-n-octylether und
a) iv) 40 - 66 Gew.-% Wasser
enthält.

9. Konditionierendes Preshave- oder Aftershavemittel nach Anspruch 7 oder 8 oder Verfahren nach einem der Ansprüche 1 bis 6 oder 8, **dadurch gekennzeichnet, dass** die Mikroemulsion a) außer den 15 - 25 Gew.-% an Alkyl(oligo)glycosid(en) keine weiteren Tenside enthält.

10. Konditionierendes Preshave- oder Aftershavemittel nach einem der Ansprüche 7 bis 9 oder Verfahren nach einem der Ansprüche 1 bis 6, 8 oder 9, **dadurch gekennzeichnet, dass** das konditionierende Preshave- oder Aftershavemittel zusätzlich zu dem mindestens einen Alkylether- oder Alkenyletherphosphat der Formel (I) als Emulgator b) i) und den Tensiden aus der Mikroemulsion a) weitere Tenside in einer Gesamtmenge von 0 bis 1,5 Gew.-%, bevorzugt 0,01 bis 1 Gew.-%, weiter bevorzugt 0,1 bis 0,3 Gew.-%, jeweils bezogen auf das Gesamtgewicht des Preshave- oder Aftershavemittels, enthält.

11. Konditionierendes Preshave- oder Aftershavemittel nach einem der Ansprüche 7 bis 10 oder Verfahren nach einem der Ansprüche 1 bis 6 oder 8 bis 10, **dadurch gekennzeichnet, dass** das Preshave- oder Aftershavemittel, jeweils bezogen auf sein Gesamtgewicht, insgesamt 1 - 7 Gew.-% Öle, bevorzugt 2 - 6 Gew.-% Öle, besonders bevorzugt 3 - 5 Gew.-% Öle, einschließlich aller mikroemulgierten und nicht mikroemulgierten Öle, enthält.

12. Konditionierendes Preshave- oder Aftershavemittel nach einem der Ansprüche 7 bis 11 oder Verfahren nach einem der Ansprüche 1 bis 6 oder 8 bis 11, **dadurch gekennzeichnet, dass** das Preshave- oder Aftershavemittel, jeweils bezogen auf sein Gesamtgewicht, insgesamt 0,05 - 5 Gew.-%, bevorzugt 0,1 - 3 Gew.-%, besonders bevorzugt 0,2 - 1,5 Gew.-% eines oder mehrerer Öle in mikroemulgierter Form enthält.

13. Konditionierendes Preshave- oder Aftershavemittel nach einem der Ansprüche 7 bis 12 oder Verfahren nach einem der Ansprüche 1 bis 6 oder 8 bis 12, **dadurch gekennzeichnet, dass** das Preshave- oder Aftershavemittel, jeweils bezogen auf sein Gesamtgewicht, insgesamt 0,95 - 6,95 Gew.-%, bevorzugt 2 - 5 Gew.-%, besonders bevorzugt 3 - 4 Gew.-%, eines oder mehrerer Öle in nicht mikroemulgierter Form enthält.

14. Nicht-therapeutische Verwendung eines konditionierenden Preshave- oder Aftershavemittels nach einem der Ansprüche 7 bis 13 zur Verbesserung des Hautgefühls und/oder zur Linderung des Spannungsgefühls und des Brennens, das als Folge der Rasur auftritt.

## Claims

1. A method for preparing a conditioning preshave or aftershave agent, comprising the following steps:
a) providing a microemulsion containing
a) i) at least one alkyl(oligo)glycoside
a) ii) at least one monoester or diester of glycerol that has at least one C₁₀-C₂₄ fatty acid as a co-emulsifier, and additionally
a) iii) at least one oil and
a) iv) water,
wherein the components (i), (ii), (iii) and (iv), in each case based on the weight of the microemulsion, are present in the amounts I, II, III and IV where
I = from 15 to 25 wt.%, based on the weight of the microemulsion,
II = from 4 to 10 wt.%, based on the weight of the microemulsion,
III = from 15 to 25 wt.%, based on the weight of the microemulsion,
IV = from 40 to 66 wt.%, based on the weight of the microemulsion, and
b) mixing the microemulsion with a hydrous cosmetic carrier, which contains:
b) i) at least one alkyl ether phosphate or alkenyl ether phosphate of formula (I) as an emulsifier, 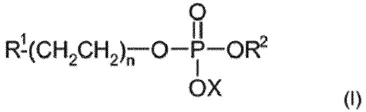 in which R¹ represents an aliphatic hydrocarbon functional group having 8 to 30 carbon atoms, R² represents hydrogen, a functional group (CH₂CH₂O)ₙR¹, or X, n represents numbers from 1 to 10, and X represents hydrogen, a functional group (CH₂CH₂O)ₙR¹, an alkali metal or alkaline-earth metal, or NR³R⁴R⁵R⁶, where R³ to R⁶ represent, independently of one another, a C₁ to C₄ hydrocarbon functional group,
b) ii) at least one oil that is different from a) iii).

2. The method according to claim 1, **characterized in that** in formula (I)
- the functional group R¹ represents at least one group selected from lauryl, myristyl, cetyl, stearyl or oleyl and mixtures thereof, in particular the mixture cetearyl,
- the functional groups R² and X represent a functional group (CH₂CH₂O)ₙR¹ and
- n represents numbers from 2 to 6, preferably 3 to 5, particularly preferably 4.

3. The method according to one of claims 1 or 2, **characterized in that** the emulsifier of formula (I) is selected from trilaureth-4 phosphate, triceteareth-4 phosphate, and mixtures thereof.

4. The method according to one of claims 1 to 3, **characterized in that** the conditioning preshave or aftershave agent contains the at least one emulsifier of formula (I) in a total amount of from 0.1 to 4 wt.%, preferably from 0.5 to 2 wt.%, more preferably from 0.8 to 1.5 wt.%, in each case based on the total weight of the preshave or aftershave agent.

5. The method according to one of claims 1 to 4, **characterized in that** the microemulsion a) contains
(i) in total from 15 to 25 wt.% of at least one alkyl(oligo)glycoside of general formula RO-[G]ₓ, in which R represents an alkyl functional group and/or an alkenyl functional group having 4 to 22 C atoms, G represents a sugar residue having 5 or 6 C atoms and x represents numbers from 1 to 10,
(ii) in total from 4 to 10 wt.% of at least one monoester and/or diester of glycerol having a saturated or unsaturated, branched or unbranched C₁₀-C₂₄ fatty acid,
(iii) in total from 15 to 25 wt.% of at least one oil that is different from a) ii) and
(iv) from 40 to 66 wt.% water,
the amounts stated relating to the weight of the microemulsion a).

6. The method according to one of claims 1 to 5, **characterized in that** the conditioning preshave or aftershave agent contains, based on the total weight thereof, from 0.5 to 50 wt.%, preferably from 1.0 to 30 wt.%, more preferably from 5.0 to 10 wt.%, of the microemulsion a).

7. A conditioning preshave or aftershave agent, containing
a) a microemulsion containing
a) i) at least one alkyl(oligo)glycoside
a) ii) at least one monoester or diester of glycerol that has at least one C₁₀-C₂₄ fatty acid as a co-emulsifier,
a) iii) at least one oil and a) iv) water,
wherein the components (i), (ii), (iii) and (iv), in each case based on the weight of the microemulsion, are present in the amounts I, II, III and IV where
I = from 15 to 25 wt.%, based on the weight of the microemulsion,
II = from 4 to 10 wt.%, based on the weight of the microemulsion,
III = from 15 to 25 wt.%, based on the weight of the microemulsion,
IV = from 40 to 66 wt.%, based on the weight of the microemulsion,
and furthermore
at least one emulsifier of formula (I) in a total amount of from 0.1 to 4 wt.%, based on the total weight of the preshave or aftershave agent, 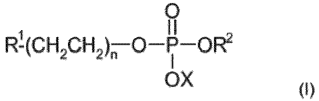 in which R¹ represents an aliphatic hydrocarbon functional group having 8 to 30 carbon atoms, R² represents hydrogen, a functional group (CH₂CH₂O)ₙR¹, or X, n represents numbers from 1 to 10, and X represents hydrogen, a functional group (CH₂CH₂O)ₙR¹, an alkali metal or alkaline-earth metal, or NR³R⁴R⁵R⁶, where R³ to R⁶ represent, independently of one another, a C₁ to C₄ hydrocarbon functional group, and b) ii) at least one oil that is different from a) iii),
wherein the preshave or aftershave agent contains, in each case based on the total weight thereof, in total from 50 to 95 wt.% water and in total from 1 to 7 wt.% oil(s).

8. The conditioning preshave or aftershave agent according to claim 7 or the method according to one of claims 1 to 6, **characterized in that** the microemulsion a), in each case based on the weight thereof, contains
a) i) from 15 to 25 wt.% C₈-C₁₆ alkyl glucosides having a degree of oligomerization of from 1.2 to 1.5,
a) ii) from 4 to 10 wt.% mixture of glyceryl monooleate and glyceryl dioleate,
a) iii) from 15 to 25 wt.% di-n-octyl ether and
a) iv) from 40 to 66 wt.%
water.

9. The conditioning preshave or aftershave agent according to claim 7 or 8 or the method according to one of claims 1 to 6 or 8, **characterized in that** the microemulsion a) does not contain any other surfactants apart from the 15 to 25 wt.% of alkyl(oligo)glycoside(s).

10. The conditioning preshave or aftershave agent according to one of claims 7 to 9 or the method according to one of claims 1 to 6, 8 or 9, **characterized in that** the conditioning preshave or aftershave agent contains, in addition to the at least one alkyl ether phosphate or alkenyl ether phosphate of formula (I) as the emulsifier b) i) and the surfactants from the microemulsion a), further surfactants in a total amount of from 0 to 1.5 wt.%, preferably from 0.01 to 1 wt.%, more preferably from 0.1 to 0.3 wt.%, in each case based on the total weight of the preshave or aftershave agent.

11. The conditioning preshave or aftershave agent according to one of claims 7 to 10 or the method according to one of claims 1 to 6 or 8 to 10, **characterized in that** the preshave or aftershave agent, in each case based on the total weight thereof, contains in total from 1 to 7 wt.% oils, preferably from 2 to 6 wt.% oils, particularly preferably from 3 to 5 wt.% oils, inclusive of all microemulsified and non-microemulsified oils.

12. The conditioning preshave or aftershave agent according to one of claims 7 to 11 or the method according to one of claims 1 to 6 or 8 to 11, **characterized in that** the preshave or aftershave agent, in each case based on the total weight thereof, contains in total from 0.05 to 5 wt.%, preferably from 0.1 to 3 wt.%, particularly preferably from 0.2 to 1.5 wt.%, of one or more oils in microemulsified form.

13. The conditioning preshave or aftershave agent according to one of claims 7 to 12 or the method according to one of claims 1 to 6 or 8 to 12, **characterized in that** the preshave or aftershave agent, in each case based on the total weight thereof, contains in total from 0.95 to 6.95 wt.%, preferably from 2 to 5 wt.%, particularly preferably from 3 to 4 wt.%, of one or more oils in non-microemulsified form.

14. The non-therapeutic use of a conditioning preshave or aftershave agent according to one of claims 7 to 13 for improving the feeling of the skin and/or for alleviating the feeling of tightness and burning that occurs as a result of shaving.

## Revendications

1. Procédé de fabrication d'un agent avant-rasage ou après-rasage revitalisant, comprenant les étapes consistant à :
a) fournir une micro-émulsion contenant
a) i) au moins un alkyl(oligo)glycoside,
a) ii) au moins un mono- ou di-ester de glycérol avec au moins un acide gras en C₁₀-C₂₄ en tant que co-émulsifiant, outre
a) iii) au moins une huile et
a) iv) de l'eau,
dans lequel les constituants (i), (ii), (iii) et (iv), rapportés respectivement au poids de la micro-émulsion, sont présents dans les quantités I, II, III et IV avec
I = de 15 à 25 % en poids, rapporté au poids de la micro-émulsion,
II = de 4 à 10 % en poids, rapporté au poids de la micro-émulsion,
III = de 15 à 25 % en poids, rapporté au poids de la micro-émulsion,
IV = de 40 à 66 % en poids, rapporté au poids de la micro-émulsion, et
b) mélanger la micro-émulsion avec un support cosmétique à base d'eau contenant :
b) i) en tant qu'émulsifiant, au moins un phosphate d'alkyléther ou d'alcényléther de Formule (I), 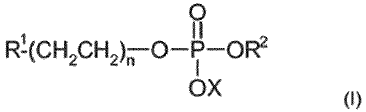 dans laquelle R¹ représente un radical hydrocarbure aliphatique comportant 8 à 30 atomes de carbone, R² représente l'hydrogène, un radical (CH₂CH₂O)ₙR¹ ou X, n représente un nombre situé dans la plage allant de 1 à 10 et X représente l'hydrogène, un radical (CH₂CH₂O)ₙR¹, un métal alcalin ou alcalino-terreux ou NR³R⁴R⁵R⁶, R³ à R⁶ représentant indépendamment les uns des autres un radical hydrocarbure en C₁ à C₄.
b) ii) au moins une huile autre que a) iii).

2. Procédé selon la revendication 1, **caractérisé en ce que**, dans la Formule (I)
- les radicaux R¹ représentent au moins un groupe choisi parmi les groupes lauryle, myristyle, cétyle, stéaryle ou oléyle et leurs mélanges, en particulier le mélange cétéaryle,
- les radicaux R² et X représentent un radical (CH₂CH₂O)ₙR¹ et
- n représente des nombres situés dans la plage allant de 2 à 6, de préférence de 3 à 5, et de façon particulièrement préférée 4.

3. Procédé selon l'une quelconque des revendications 1 et 2, **caractérisé en ce que** l'émulsifiant de Formule (I) est choisi parmi le trilaureth-4-phosphate, le tricétéareth-4-phosphate et leurs mélanges.

4. Agent cosmétique selon l'une quelconque des revendications 1 à 3 **caractérisé en ce que** l'agent avant-rasage ou après-rasage revitalisant contient l'au moins un émulsifiant de Formule (I) en une quantité totale située dans la plage allant de 0,1 à 4 % en poids, de préférence de 0,5 à 2 % en poids, de façon particulièrement préférée de 0,8 à 1,5 % en poids, rapportée au poids total de l'agent avant-rasage ou après-rasage.

5. Procédé selon l'une quelconque des revendications 1 à 4, **caractérisé en ce que** la micro-émulsion a)
(i) contient un total de 15 à 25 % en poids d'au moins un alkyl(oligo)glycoside de formule générale RO-[G]x, où R représente un radical alkyle et/ou alcényle ayant de 4 à 22 atomes de carbone, G représente un radical de sucre ayant 5 ou 6 atomes de carbone, et x représente un nombre situé dans la plage allant de 1 à 10,
(ii) un total de 4 à 10 % en poids d'au moins un mono-ester et/ou di-ester de glycérol avec un acide gras en C₁₀-C₂₄ saturé ou insaturé, ramifié ou non ramifié,
(iii) un total de 15 à 25 % en poids d'au moins une huile autre que a) ii) et
(iv) de 40 à 66 % en poids d'eau,
les indications de quantités se rapportant chacune au poids total de la micro-émulsion a).

6. Procédé selon l'une quelconque des revendications 1 à 5, **caractérisé en ce que** l'agent avant-rasage ou après-rasage revitalisant contient, rapporté à son poids total, de 0,5 à 50 % en poids, de préférence de 1,0 à 30 % en poids, de façon particulièrement préférée de 5,0 à 10 % en poids, de la micro-émulsion a).

7. Agent avant-rasage ou après-rasage revitalisant contenant
a) une micro-émulsion contenant
a) i) au moins un alkyl(oligo)glycoside,
a) ii) au moins un mono- ou di-ester de glycérol avec au moins un acide gras en C₁₀-C₂₄ en tant que co-émulsifiant,
a) iii) au moins une huile et
a) iv) de l'eau,
dans lequel les constituants (i), (ii), (iii) et (iv), rapportés respectivement au poids de la micro-émulsion, sont présents dans les quantités I, II, III et IV avec
I = de 15 à 25 % en poids, rapporté au poids de la micro-émulsion,
II = de 4 à 10 % en poids, rapporté au poids de la micro-émulsion,
III = de 15 à 25 % en poids, rapporté au poids de la micro-émulsion,
IV = de 40 à 66 % en poids, rapporté au poids de la micro-émulsion,
outre
au moins un émulsifiant de Formule (I) en une quantité totale située dans la plage allant de 0,1 à 4 % en poids, rapportée au poids total de l'agent avant-rasage ou après-rasage, 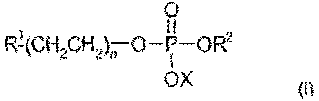 où R¹ représente un radical hydrocarboné aliphatique ayant de 8 à 30 atomes de carbone, R² représente un atome d'hydrogène, un radical (CH₂CH₂O)ₙR¹ ou X, n représente un nombre situé dans la plage allant de 1 à 10 et X représente un atome d'hydrogène, un radical (CH₂CH₂O)ₙR¹, un métal alcalin ou alcalino-terreux ou NR³R⁴R⁵R⁶, R³ à R⁶ représentant indépendamment les uns des autres un radical hydrocarboné en C₁ à C₄, et b) ii) au moins une huile autre que a) iii), dans lequel l'agent avant-rasage ou après-rasage contient - rapporté à son poids total - un total de 50 à 95 % en poids d'eau et un total de 1 à 7 % en poids d'huile(s).

8. Agent avant-rasage ou après-rasage revitalisant selon la revendication 7 ou procédé selon l'une quelconque des revendications 1 à 6, **caractérisé en ce que** la micro-émulsion a) contient, rapportés respectivement à son poids,
a) i) de 15 à 25 % en poids d'alkylglucoside en C₈-C₁₆présentant un degré d'oligomérisation situé dans la plage allant de 1,2 à 1,5,
a) ii) de 4 à 10 % en poids d'un mélange de mono-oléate de glycéryle et de di-oléate de glycéryle,
a) iii) de 15 à 25 % en poids de di-n-octyléther et
a) iv) de 40 à 66 % en poids d'eau.

9. Agent avant-rasage ou après-rasage revitalisant selon la revendication 7 ou 8, ou procédé selon l'une quelconque des revendications 1 à 6 ou 8, **caractérisé en ce que** la micro-émulsion a) ne contient, outre les 15 à 25 % en poids d'alkyl(oligo)glycoside(s), aucun autre tensioactif.

10. Agent avant-rasage ou après-rasage revitalisant selon l'une quelconque des revendications 7 à 9, ou procédé selon l'une quelconque des revendications 1 à 6, 8 ou 9, **caractérisé en ce que** l'agent avant-rasage ou après-rasage revitalisant contient, en plus de l'au moins un phosphate d'alkyléther ou d'alcényléther de Formule (I) en tant qu'émulsifiant b) i) et des tensioactifs de la micro-émulsion a), d'autres tensioactifs en une quantité totale située dans la plage allant de 0 à 1,5 % en poids, de préférence de 0,01 à 1 % en poids, de façon particulièrement préférée de 0,1 à 0,3 % en poids, rapportée au poids total de l'agent avant-rasage ou après-rasage.

11. Agent avant-rasage ou après-rasage revitalisant selon l'une quelconque des revendications 7 à 10, ou procédé selon l'une quelconque des revendications 1 à 6, ou 8 à 10, **caractérisé en ce que** l'agent avant-rasage ou après-rasage contient, rapporté à son poids total, un total de 1 à 7 % en poids d'huiles, de préférence de 2 à 6 % en poids d'huiles, de façon particulièrement préférée de 3 à 5 % en poids d'huiles, en ce compris toutes les huiles micro-émulsionnées et non micro-émulsionnées.

12. Agent avant-rasage ou après-rasage revitalisant selon l'une quelconque des revendications 7 à 11, ou procédé selon l'une quelconque des revendications 1 à 6, ou 8 à 11, **caractérisé en ce que** l'agent avant-rasage ou après-rasage contient, rapporté à son poids total, un total de 0,05 à 5 % en poids, de préférence de 0,1 à 3 % en poids, de façon particulièrement préférée de 0,2 à 1,5 % en poids d'une ou plusieurs huile(s) sous forme micro-émulsionnée.

13. Agent avant-rasage ou après-rasage revitalisant selon l'une quelconque des revendications 7 à 12, ou procédé selon l'une quelconque des revendications 1 à 6, ou 8 à 12, **caractérisé en ce que** l'agent avant-rasage ou après-rasage contient, rapporté à son poids total, un total de 0,95 à 6,95 % en poids, de préférence de 2 à 5 % en poids, de façon particulièrement préférée de 3 à 4 % en poids d'une ou plusieurs huile(s) sous forme non micro-émulsionnée.

14. Utilisation non-thérapeutique d'un agent avant-rasage ou après-rasage revitalisant selon l'une quelconque des revendications 7 à 13 pour améliorer le confort cutané et/ou soulager la sensation de tiraillement et de brûlure causée par le rasage.
